(19) Europäisches Patentamt
European Patent Office
Office européen des brevets

(11) Publication number: **0 343 955 A2**

(12) # EUROPEAN PATENT APPLICATION

(21) Application number: **89305247.2**

(22) Date of filing: **24.05.89**

(51) Int. Cl.[4]: **C 12 Q 1/68**
G 01 N 33/542

(30) Priority: **27.05.88 US 199655**

(43) Date of publication of application:
**29.11.89 Bulletin 89/48**

(84) Designated Contracting States:
**AT BE CH DE ES FR GB GR IT LI LU NL SE**

(71) Applicant: **BIOVENTURES, INC.**
**848 Scott Street**
**Murfreesboro Tennessee 37129 (US)**

(72) Inventor: **Dawson, Elliott P.**
**Box 85 Maple Street**
**Bell Buckle Tennessee 37020 (US)**

(74) Representative: **Jump, Timothy John Simon et al**
**VENNER, SHIPLEY & CO. 368 City Road**
**London EC1V 2QA (GB)**

(54) **Process for complex binding of targeted molecular species.**

(57) A method, targeting selected molecular species to produce at least one selected substance in a fluid including conjugating a first label to a first moiety and a second label to a second moiety, the first and second moieties complimentarily binding to complimentary entities being one of, 1) respective first and second complimentary sites on a solid phase, and 2) each other, and 3) a single entity, complimentary to both; and producing a selected substance, the substance being produced in the presence of a first substrate, by an interaction between the first label and the second label, the amount of the substance being enhanced by the close proximity of the labels. The method may, for example, be an assay to determine the presence or concentration of at least one selected substance in a fluid. The method may also, for example, produce a toxigenic complex on a cell membrane in a fluid.

EP 0 343 955 A2

## Description

# PROCESS FOR COMPLEX BINDING OF TARGETED MOLECULAR SPECIES

The present invention relates to a process for complex binding of targeted molecular species. In particular, the present invention relates to certain molecular interactions of targeted molecular species and their use in molecular identification and production of useful end products.

Specific interactions between molecules are well known in biochemistry and permit the construction of many types of analytical techniques. Typically in a biological sample the molecule or compound of interest is found to reside in a highly complex matrix containing many molecules of different types and even some molecules very similar in structure to the molecule of interest. The detection of the species of interest is complicated not only by the presence of other substances but also by the fact that the species of interest may be present in only trace amounts. Consequently assays for measuring or detecting species of interest have been developed which are highly specific for the species of interest and sensitive with respect to being able to indicate the presence of only trace amounts of the species of interest.

There exists a number of conventional techniques for detection of species of interest in biological specimens. These methods include antigen and antibody interactions such as agglutination reactions, gel diffusion reactions,complement fixation, immunoelectrophoresis, western blotting, fluorescent antibody techniques, radio immunoassay, sandwich enzyme-linked immunosorbent assays, and enzyme modulation immunoassays; receptor-modulator assays such as receptor assays and modulator-effector molecule assays; cell surface marker assays such as HLA typing assays, receptor assays, cell surface antigen assays, tumor marker assays; specific protein assays such as dot blotting, and binding assays; specific complex carbohydrate assays such as lectin binding; and specific nucleotide sequence homology assays such as complimentary DNA or RNA probes, Southern blotting, Northern blotting, kinetic assays, in situ probes. Davis, Leonard G., Dibner, Mark D., Batley, James F., Basic Methods in Molecular Biology (Elsevier, New York) 1986.

Immunologically based methods have broad applicability in a number of significant areas of research and diagnostic procedures. Immunologically based assays derive their specificity from the specific interaction of the paratope of the antibody and the epitope of the antigenic species. The ability to distinguish between either bound or free antigen or antibody is fundamental to these methodologies. The sensitivity of these assays is dependent upon the method incorporated into the assay to permit distinguishing either free or bound forms of the antigens or the antibody. The assays can be configured to measure the species of interest directly or indirectly or by means of competition with a labelled species. The speed with which these assays may be accomplished is dependent upon the kinetics of binding between antigen and antibody and their respective affinities for one another, as well as their relative concentrations to one another and their concentrations within the sample matrix.

An immunoassay employing enzyme channeling includes an enzyme participating in a coupled enzymatic reaction which is immobilized to a solid phase. Litman, D. J., Hanlon, T. M., and Ullman, E. F., Anal. Biochem 106: 223 (1980). To the same solid phase, an antigen is also immobilized independently of the enzyme. The other enzyme participating in the coupled enzymatic reaction is conjugated with antibodies reactive with the antigen immobilized to the solid phase.

The binding of labelled antibody to antigen causes the partitioning of one of the labelled enzymes between a catalytically inactive bulk phase and a catalytically active microenvironment at the solid phase. Channeling is said to occur when the enzymes catalyzing the coupled reactions are brought together via an antibody-antigen binding event at a surface or in a microenvironment where diffusion controlled exchange with the bulk solution is limited. Gibbons, I., Armenta, R., DiNello, R., and Ullman, E. F., "Nonseparation Enzyme Channeling Immunometric Assays", Methods in Enzymology. 136: 93-103 (Academic Press, New York) 1983. The diffusion of a first product away from the microenvironment and into the bulk phase is limited and establishes a local concentration of the product within the microenvironment that exceeds that in the bulk solution. In the enzyme channeling immunoassay only one of the binding species is conjugated to a label thus providing limited flexibility in binding enzymes. In the present invention the coupled enzymes are each directly conjugated to the species which undergo the binding reaction.

The enzyme channeling assay is further limited by depending on the establishment of local concentrations of the first product, different from that in bulk solution. In the present invention there is no significant difference in the concentration of a first product in either its bound form or in free solution. The present invention has a superior rate of reaction due to the closeness of a sequential enzyme and to its substrate. The present invention possesses superior flexibility in targeting selected molecular species and superior in efficiencies due to the different techniques of bringing, for example, enzymes together.

Receptor-modulator assays also have applicability to research and diagnostic procedures. Receptors are typically large molecules unique to a cell type, which extend beyond the cell membrane into the extracellular matrix. The extracellular component of the receptor has the specific ability to bind a molecule or molecules possessing a unique molecular structure. The bound molecule is usually referred to as the modulator and the specificity of binding may be so narrow as to exclude any other molecule from binding to the receptor except for the modulator, or so broad that retention of only modest features of the modulator are required for binding, thereby permitting many other similar species to bind to the receptor. The interaction between modulator and receptor usually has a regulatory role in the biological processes and functionality of the cells on which it is found. However, certain species (for example, drugs and synthetic or naturally derived peptides or other molecules) not otherwise endogenous to the in vivo conditions, may also exhibit binding to a receptor. Advances Biochemical Psychopharmacology, "Molecular Pharmacology of Neurotransmitter Receptors", Tomio Segawa, Henry I. Yamamera, Kinya Kuriuama, Edts., Vol 36 (Raven Press, New York) 1983. Methods for measuring receptors and their modulators or other species which bind to the receptor depend upon distinguishing between bound and free forms of receptors or modulator. O'Brien R.D. "Problems and Approaches in Non-Catalytic Biochemistry" in The Receptors: A Comprehensive Treatise, Vol I General Principals and Procedures R.D. O'Brien, Edts., (Plenum Press, New York) 1979. The methods may be direct or indirect or competitive in nature. The ability to detect the interaction between a receptor and its modulator is similarly dependent upon the conditions as described above for antibody and antigen binding. When receptors are measured on intact cells or by the isolation of receptors, detection of the receptor may be dependent upon the developmental stage of the cells from which it is isolated and upon the phase of the cell-cycle for the cells bearing the receptor of interest. Synder,S.H., Chang, K.J., Kwham, M.J. and Yamerra, H.I. "Biochemical Identification of the Mammalian Muscarinic Chloenergic Receptors", Fed. Proc., 34: 1915-1921 (1975).

Cell surface markers are distinct from cell receptors in that they appear on the surface of a cell type and are unique to that type. They may or may not have a regulatory role or their precise role may be undetermined. In some tumors the markers may represent aberrations of cellular function characteristic of the oncogenic state, as certain markers are associated with certain tumors and are shed into the extracellular matrix. The measurement of the levels of certain markers is used to establish a prognosis in post surgical patients and the reappearance or increase in the levels of markers is associated with relapse and reappearance of the tumors. Tumor markers are associated with the expression of oncogenic genes or with developmental immaturity similar to the embryonic state. Assays to measure carcinoembryonic antigen, alpha feto protein and neuron specific enolase and prostate specific antigen are in use to develop a prognosis for affected patients. Monoclonal Antibodies in Clinical Medicine J. Fabre and A. McMichael, Edts., (Academic Press, London) 1982.

Specific protein assays or assays for other moieties such as polysaccharide, DNA, RNA, lipo proteins, glycosylated proteins and their fragments or synthetic constructs can be detected because of the specific affinity they may possess for other species. Assays are employed to detect the presence or absence of either the binding entity or the bound entity and may be employed in such a manner as to reveal the quantity present of either entity. Binding assays and blotting assays are typically employed to perform the measurement. Johnson, D.A., Gautsch, J.W. Sportsman, J.R., and Elder, J.H., Gene Anal. Tech. 1: 3 1984; Towbin, H.H., Staehelin, T, and Gordon, J., Proc. Natl. Acad. Sci. USA. 76: 4350, 1979; Hawkes, R. et al, Analytical Biochemistry 119: 142-147 (1982).

Lectins, are sugar binding, cell-agglutinating proteins of non-immune origin from plants, animals or microorganisms. They are widely used in biochemical research to indicate the presence or absence of specific carbohydrate moieties in materials of biological origin. Halena Lis and Nathan Sharon "Lectins as Molecules and Tools in Annual Reviews in Biochemistry 55: 35-67 (1986); Leiner, I.E., Sharon, N. and Goldstein, I.J., Edts., The Lectins: Properties, Functions and Applications in Biology and Medicine, (Academic Press, New York) 1986.

Specific nucleotide sequence assays depend upon the binding of detectable complimentary DNA (cDNA) or complimentary RNA (cRNA) to probe regions of single stranded DNA or RNA suspected to contain a nucleotide sequence capable of binding the detectable probe. Such techniques are employed to establish the identity of an organism, the presence of a gene sequence, or the efficiency of gene integration in the construction of recombinant organisms. Techniques forming the basis for this assay are Southern blotting, Northern blotting, kinetic association assays, and in situ probing. Southern, E.M. Journal of Molecular Biology 98: 503517, 1975; Wahl G. ,Stein, M. and Stark, G., Proc. Natl. Acad. Sci. USA 76: 3683, 1979; Lehard, H., Diamond, D., Wozney, J. and Boedtker, H., Biochemistry 16: 4743, 1977; Thomas, P., Proc. Natl. Acad. Sci. USA. 77: 5201, 1980; Kafatos, F.C., C.W. Jones and A. Efstratiadas Nucleic Acids Research 7: 1541-1552, 1979.

A method capable of accomplishing any of the above assays more rapidly and with greater precision are needed by researchers and clinicians. Such new method will provide a significant benefit to researchers and clinicians by enabling them to obtain more accurate results in a more timely and efficacious manner. The present invention provides such a new method.

In accordance with the presently preferred exemplary embodiments, a method is provided for the use or determination of molecular species capable of exhibiting specific binding to one another, jointly and simultaneously or individually and separately.

The useable, detectable and measurable types and varieties of specific interactions embraced by the invention are applicable to any and all specific interactions between moieties by means of detectable labelling achieved by means of linking two enzymes each of which catalyzes one of the reactions of a sequential set of reactions. One enzyme of which is linked to one set of one or the other or both moieties of interest, and the other enzyme of the sequential pair is linked to another set of one or the other or both of the moieties of interest, and that upon binding occurring between the moieties of interest the enzymes are brought into molecular proximity with one another. By the measurement of the rate of reaction (kinetic method) or by the accumulation of product until the reaction is quenched (end-point method), the presence or the quantity of either moiety may be determined.

When enzymes are employed as the conjugates they must be capable of catalyzing a reaction. The progress of which can be measured, or the products of which may be detected by subsequent reaction when the two enzymes are employed as labels, the reaction which is measured is that catalyzed by the second label or the subsequent reaction of its products. The progress of the reaction or the subsequent reaction of its products may be measured by endowing detection after the reaction has been quenched, or preferably by a kinetic method in which the accumulation of reaction products is measured over time. Possible pairs of enzyme labels for ascertaining the activity of bound labels include luciferase from Photobacterium fischeri, (E.C. 1.14.14.3) and NAD(P)H:FMN oxidoreductase (E.C. 1.6.8.1) and the pair D-amino acid oxidase (E.C. 1.4.3.3) and lactoperoxidase (E.C. 1.11.7.) and the pair B-galactosidase (E.C. 3.2.1.23) and B-galactose dehydrogenase (E.C. 1.1.1.48)..

The luciferase from Photobacterium fischeri catalyzes the reaction (oxidation) of the reduced flavin nucleotide (FMNH2) and an aldehyde (long chain) by molecular oxygen yielding the oxidized flavin mononucleotide (FMN), the corresponding acid of the aldehyde, water and light (Brolin, S.E. et Al Anal Biochem 42, 124 (1971).

The NAD(P)H:FMN oxidoreductase from the same organism catalyzes the oxidation of NAD(P)H to NAD(P) with the consequent reduction of FMN to FMNH2 The oxidoreductase may be summarized as follows:

$2NAD(P)H + FMN \text{-------->} 2NAD(P)^+ + FMNH_2$

The luciferase reaction may be summarized as follows:

$FMNH_2 + RCHO + O_2 \text{-------->} FMN + RCO_2H + H_2O + .2hv$ 495nm

When the two reactions are coupled the NAD(P)H reduces the FMN to $FMNH_2$ which permits the bioluminescent reaction to take place. The reaction is monitored by the quantization of light emitted at 495nm.

D-amino acid oxidase catalyzes the oxidative deamination of D-amino acids to the corresponding Beta-keto acids. The reaction may be written as follows:

$R\text{-}CNH_2COOH + O_2 + H_2O \text{-------->} RCOCOOH + NH_3 + H_2O_2$

Lactoperoxidase catalyzes the oxidation of a large number of electron donors particularly phenolic compounds and aromatic amines. The reaction may be written as follows:

e-donor + $H_2O_2$ --------> oxidized donor + $2H_2O$

When the reactions catalyzed by D amino acid oxidase and lactoperoxidase are coupled, the net result is that the hydrogen peroxide produced by the action of DAAO serves as a substrate for lactoperoxidase. The activity of the coupled enzymes is measured by the rate of formation or accumulation of oxidized donor.

B-galactosidase catalyzes the hydrolysis of the dissacharide lactose into B-D-galactose and D-glucose. The reaction may be written as follows:

$H_2O$+ lactose --------> B-D-galactose + D-glucose

B-D galactose dehydrogenase catalyzes the oxidation of B-D-galactose to D-galactino-$\delta^1$-lactone and NAD+ is reduced to NADH. The reaction may be written as follows:

B-D-galactose + $NAD^+$ -------->D-galactino-$\delta^1$-lactone + NADH + $H^+$

When the reactions catalyzed by B-galactosidase of B-galactose dehydrogenase are coupled the reaction is measured by determining the amount of NADH formed in the course of the reaction. Very high sensitivity for this reaction can be obtained by a modification of the method of enzyme cycling described by Lowry and Passoneau (A Flexible System of Enzymatic Analysis, Academic Press, New York (1972)).

The NADH produced in the reaction is coupled to the action of diaphorase in the presence of a phenazine dye which is reduced to a formazan dye which absorbs light in the visible range of the spectrum. The coupling of the reaction to diaphorase serves to regenerate $NAD^+$ and each cycle gives a net increase in the amount of formazan dye produced in each cycle of NADH -> $NAD^+$.

Suitable moieties for conjugation with label must be of maximum purity and specificity. Unconjugated label and moiety are separated from the labeled moiety by gel filtration chromatography or dialysis, depending on the molecular weight of the label. The label must also be of maximum purity; if enzymes are employed as labels, they must be of the maximum purity and specificity reasonably obtainable. The conjugation of the moieties with label is made using techniques known in the prior art. Conjugates may, for example, be enzymes or fluorescent species. In the case of enzyme conjugates, see Methods in Enzymology, Vol. 37, p 133 (1975). In the case of fluorescent conjugates, see N.B. Cherry et Al., Staining Technologies 44, 179 (1962).

In the present invention, half of the moieties are conjugated with label 1 and the other half are separately conjugated with label 2. The moieties should be capable of reaction with one another and be purified.

If an enzyme is employed as the conjugate, it must be capable of catalyzing a reaction, the progress of which can be measured, or the products of which may be detected by subsequent reaction. When two enzymes are employed as labels, the reaction which is measured is that catalyzed by the second label or the subsequent reaction of its products. The progress of the enzyme reaction or the subsequent reaction of its products may be measured by end point detection after the reaction has been quenched, or preferably by a kinetic method in which the accumulation of reaction products is measured over time.

The present invention further includes the ability to produce local levels of toxins in the region proximate to the bound enzymes catalyzing a sequential interaction resulting in predetermined cytotoxicity.

For the purposes herein moieties are entities or substances, both man made or natural, the derivatives there of or fragments thereof, being capable of exhibiting binding of a specific or categorical nature either as the bound species or the binding species or both. Enzymes may also be any reactive center, naturally occurring or man made, their fragment, derivatives or synthetic or chimeric constructs, causing, facilitating or party to the sequential reactions described.

The invention also embraces the use of haptenizable compounds, peptides, anti-idiotype antibodies, man made substances, naturally occurring substances, their fragments or derivatives or chimeric constructs, where antigens are employed in the foregoing descriptions.

The invention also embraces the use of anti-idiotype antibodies, antibodies and monoclonal antibodies, their fragments ($Fab_2$, Fab and reductive fragments obtained by the action of 2-mercaptoethyl amine), synthetic or chimeric constructs whether naturally occurring or man made and their fragments or derivatives, where antibodies are employed in the foregoing descriptions.

The invention also embraces the inversion of the labels, that is the use of label 1 for label 2 and the use of label 2 for label 1 for conjugation to the binding species.

The invention embraces the described methods of detection in any type of matrix.

Fundamental embodiments of the invention are described as follows:

DIAG. 1. is a general schematic depicting the principles of the present invention.

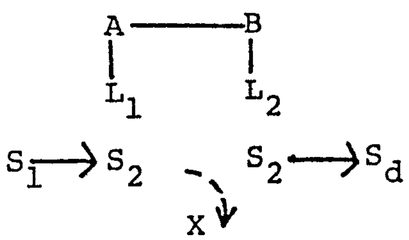


Where $A-L_1$ symbolizes a bindable moiety conjugated to label 1;
$B-L_2$ symbolizes an entity capable of binding $A-L_1$, conjugated to label 2;
$S_1$ symbolizes substrate acted on by label 1;
$S_2$ symbolizes substrate produced by label 1, and acted upon by label 2;
$S_d$ symbolizes the detectable signal produced by the action of label 2 on $S_2$;
X symbolizes a scavenger capable of acting on $S_2$.

<u>Method For Detecting Or Measuring An Antigen Or Antibody To The Antigen Jointly And Simultaneously Or Individually And Separately</u>

DIAG.2. is a schematic representation of the methods for detecting or measuring an antigen or an antibody to the antigen jointly and simultaneously or individually and separately.

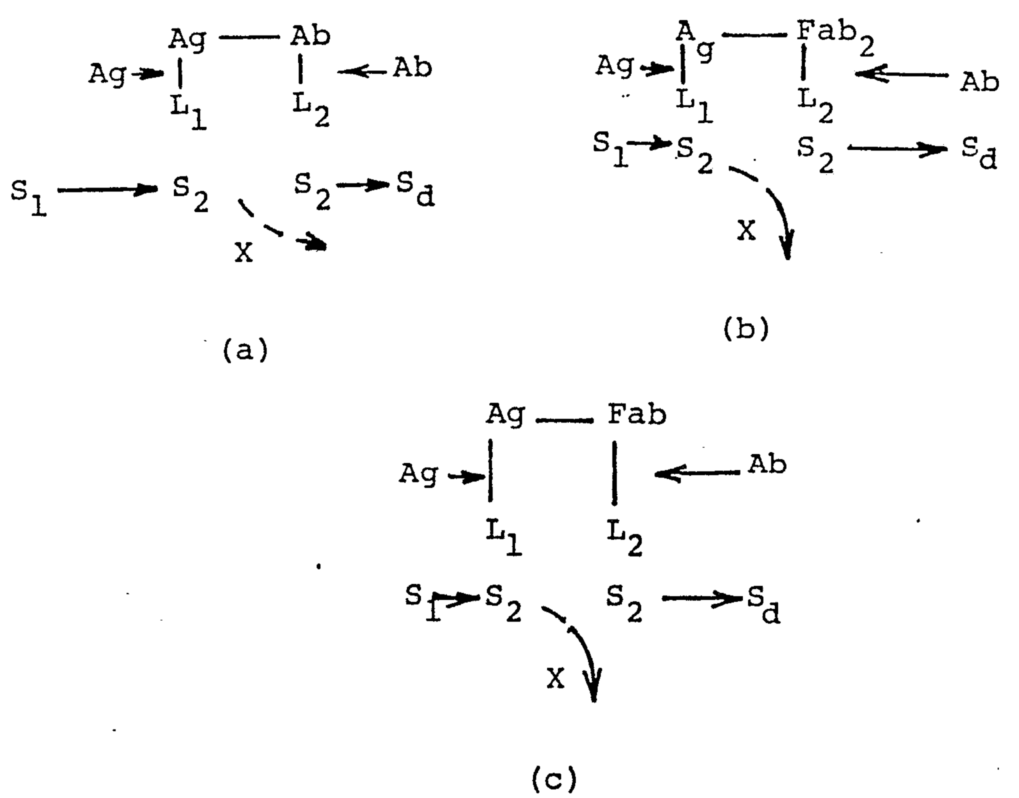

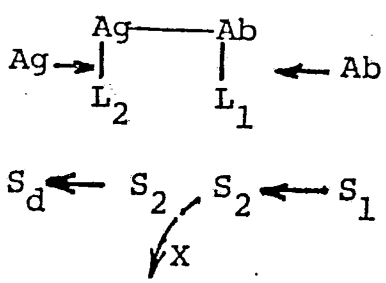

(d)

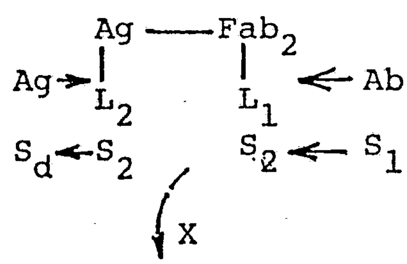

(e)

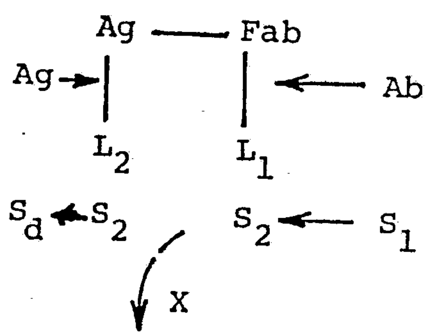

(f)

Where Ab-> symbolizes unlabelled antibody in competition with Ab-$L_2$ for binding to Ag-$L_1$;
Ag-> symbolizes unlabelled antigen in competition with Ag-$L_1$ for binding to Ab-$L_2$;
$Fab_2$ symbolizes $Fab_2$ fragment of antibody;
Fab symbolizes Fab fragment of antibody;
Ag-$L_1$ symbolizes antigen conjugated to label 1;
Ag-$L_2$ symbolizes antigen conjugated to label 2;
Ab-$L_1$ symbolizes antibody conjugated to label 1;
Ab-$L_2$ symbolizes antibody conjugated to label 2;
$Fab_2$-$L_1$ symbolizes $Fab_2$ conjugated to label 1;
$Fab_2$-$L_2$ symbolizes $Fab_2$ conjugated to label 2;
Fab-$L_1$ symbolizes Fab conjugated to label 1;
Fab-$L_2$ symbolizes Fab conjugated to label 2;
$S_1$ symbolizes substrate acted upon by label 1;
$S_2$ symbolizes substrate produced by label 1 and acted upon by label 2;
$S_d$ symbolizes the detectable signal produced by the action of label 2 on $S_2$;
X symbolizes a scavenger capable of acting on $S_2$.

According to the proximate binding technique, antigen conjugated with the first label (Ag-$L_1$) available to bind to antibody conjugated with the second label (Ab-$L_2$) is incubated in the presence of free antigen or antibody. The competition for available binding sites causes the displacement of the labelled entity The signal produced by the interaction of the first label and the second label produces a detectable signal ($S_d$). This signal is generated by the action of the first label on a first substance ($S_1$), followed by the action of the second label on a second substrate ($S_2$), which was produced in the first reaction. Accordingly the quantity, rate or quality of the signal produced is altered by the presence of the first label which is bound in proximity to the second label. The interaction of the first and second labels conjugated to unbound antigen and antibody in solution is such that the detectable signal caused by such interaction is minimal; any substrate produced by the unbound first label may be scavenged from the solution by the action of a scavenger (X) to prevent its reaction with the second label. The amount of signal produced is dependent upon the labels acting in concert.

In the presence of either free unlabelled antibody the amount of signal produced is diminished from that permitted in the absence of free unlabelled antigen or free unlabelled antibody. The amount of signal reduction is quantitatively and qualitatively related to the amount of the unlabelled entity present in the specimens.

Detecting the presence of antigen

Free unlabelled antigen (Ag->) competes with antigen conjugated to the first label (Ag-L1) for binding with antibody conjugated to the second label (Ab-L2). The diminution in signal (Sd) from that achievable in the absence of unlabelled antigens is related to the amount of unlabelled antigen in the specimen and is a measure of the quantity of unlabelled antigen present in the specimen.

Detecting the presence of antibody

Free unlabelled antibody (Ab->) competes with antibody conjugated to the first label (Ab-L1) for binding with antigen conjugated to the second label (Ag-L2). The diminution in signal (Sd) from that achievable in the absence of unlabelled antibody is related to the amount of unlabelled antibody in the specimen and is a measure of the quantity of unlabelled antibody present in the specimen.

Simultaneously detecting the presence of either antigen or antibody

Free unlabelled antigen (Ag->) or antibody (Ab->) competes with its labelled counterpart (Ag-> with Ag-L1 and Ab-> with Ab-L2) for binding of the conjugated moieties in proximity to one another. The overall diminution in signal (Sd) from that achievable in the absence of unlabelled antigen or antibody is related to the amount of unlabelled antibody or antigen in the specimen and is a measure of the quantity of unlabelled antigen or antibody or both present in the specimen and is an indicator of the presence of either.

EXAMPLE: Antigenic peptide from GP 120 of HTLV-III is reacted with sulfosuccinmidyl 4-(N-maleimideomethyl) cyclohexane-1-carboxylate (Sulfo-SMCC) to yield maleimide peptide. The maleimide peptide is reacted with the free sulfhydryl groups of urease (EC 3.5.1.5) created by the reaction of urease with 2-iminothiolane (Trout's reagent) to produce a peptide-urease conjugate. Antibody, Fab2, Fab or reduced antibody fragments produced using 2-mercaptoethyl amine are conjugated to alkaline phosphatase (E.C. 3.1.3.1).

Stoichiometrically equivalent amounts of the peptide-urease conjugate and the antibody- alkaline phosphatase conjugate are combined in the presence of specimen containing either viral antigen GP 120 from HTLV-III or antibodies produced in response to HTLV-III infection. Urea and p-nitrophenyl phosphate substrate is added. The presence of either antigen, antibody or both in the specimen causes a decrease in the observed signal from that produced in the absence of unlabeled antigen, antibody or both being in the specimen. Levamisole may be added to inhibit alkaline phosphatase activity in the specimen sample. The reaction may be measured by kinetic or by endpoint methods at 405nm for absorbance. A weakly acidic buffer may be employed to scavenge the ammonia produced by the reaction of urease on urea in free solution. The conjugates may be inverted, i.e., alkaline phosphatase-peptides and urease-antibody conjugate may be prepared.

Solid Phase Method For Detecting Or Measuring An Antigen Or Antibody To The Antigen Separately

DIAG. 3. is a schematic representation of solid phase methods for detecting or measuring an antigen or antibody to the antigen separately.

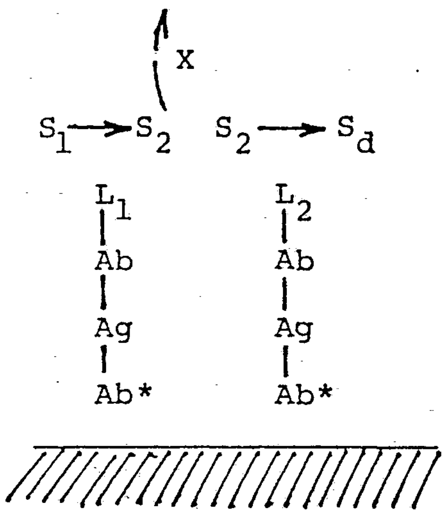
X
S1 → S2 S2 → Sd
L1
Ab
Ag
Ab*
L2
Ab
Ag
Ab*

(a)

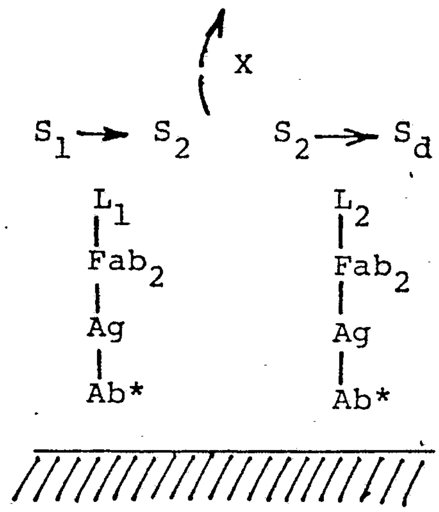
X
S1 → S2 S2 → Sd
L1
Fab2
Ag
Ab*
L2
Fab2
Ag
Ab*

(b)

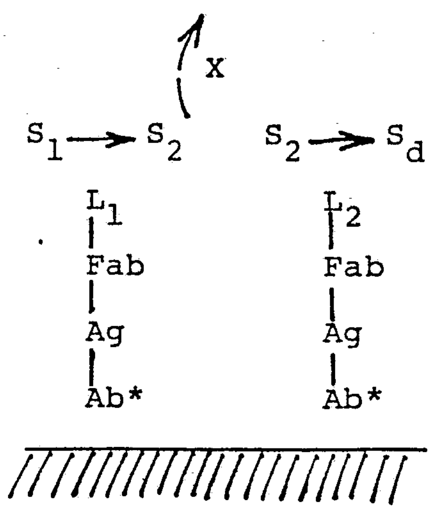
X
S1 → S2 S2 → Sd
L1
Fab
Ag
Ab*
L2
Fab
Ag
Ab*

(c)

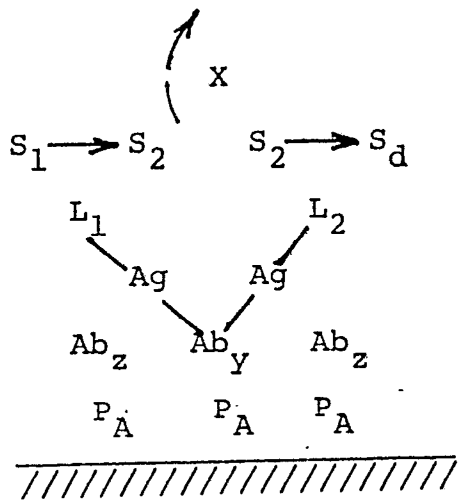


(d)

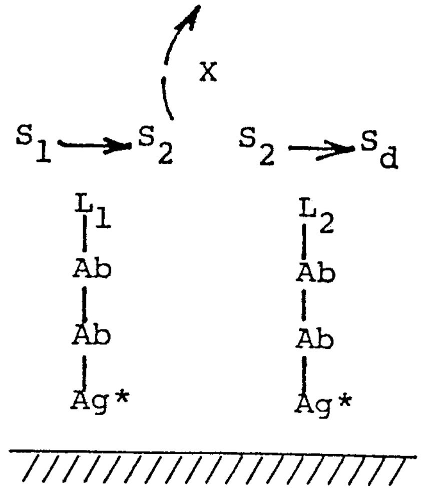


(e)

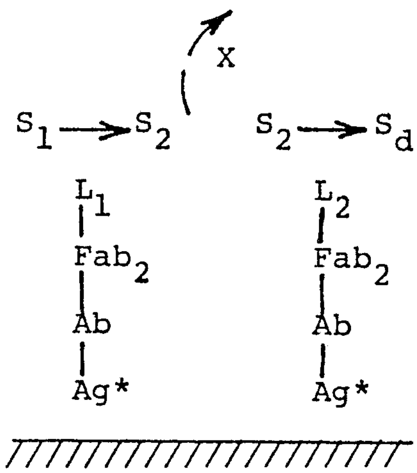


(f)

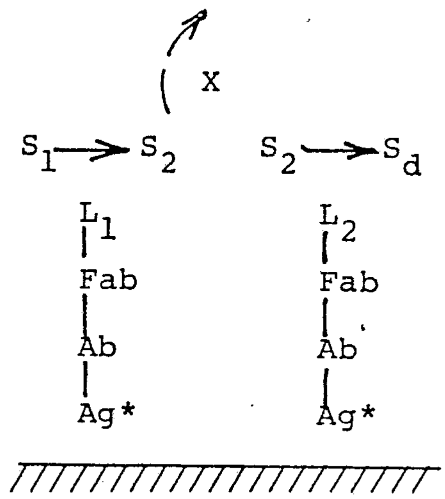


(g)

Where the symbols have the same meaning as in DIAG. 2. and,
Ag* symbolizes antigen bound to a solid phase;
Ab* symbolizes antibody bound to a solid phase;
//// symbolizes the solid phase;
$P_A$ symbolizes protein A;
$Ab_y$ symbolizes antibodies reactive with antigen;
Abz symbolizes antibodies nonreactive with antigen.

Detection of antigens: DIAG. 3(a)-(c).

Unlabelled antibodies (Ab*) against the antigen of interest are bound to a solid phase (microtiter plate, dipstick, membrane filter matrix). Antigen (Ag) containing specimen is incubated in the presence of the unlabelled antibodies for a sufficient length of time to accomplish binding. Unbound antigen may or may not be required to be removed from the matrix by washing.

Two sets of antibodies, each against the antigen of interest, one set conjugated to label 1 ($Ab\text{-}L_1$) and the other set conjugated to label 2 ($Ab\text{-}L_2$) are permitted to bind with antigen bound to the solid phase bound antibody. The formation of the ternary complex comprised of [$Ab\text{-}L_1$-($Ab\text{-}L_2$)-Ag-Ab* solid phase] which brings the labels into proximity with one another. The signal producing reaction is significantly greater in the ternary complex as opposed to that occurring in free solution between the labels. The amount of signal ($S_d$) is proportional to the amount of antigen present in the sample and is a measure of the concentration of the antigen in the specimen. A scavenger (X) may be added to remove $S_2$ produced by the action of label 1 in free solution in order to diminish the background signal. Unbound labelled antibodies may be removed by washing prior to the addition of substrate to reduce background signal.

EXAMPLE: Antibodies, $Fab_2$, Fab or reductive fragments of the antibody, reactive with HTLV-III antigens are immobilized on a solid phase (microtiter or membrane). Conjugates of antibody $Fab_2$, Fab or reductive

fragments of the antibody reactive to HTLV-III are conjugated to urease and alkaline phosphatase as previously described.

Specimen containing HTLV-III antigens are reacted with the solid phase bound antibody. Unbound specimen may be removed by washing but is not essential. Stoichiometrically equivalent amounts of the two antibody-enzyme conjugates are added and allowed to bind. Unbound conjugates may be removed by washing but this is not essential. Urea and p-nitrophenyl phosphate substrates are added. The presence of HTLV-III antigen is indicated by the increase in absorbance at 405nm from that obtained in the absence of HTLV-III antigens. Weakly acidic buffer may be employed to scavenge ammonia produced by the action of urease on urea in free solution. Levamisole may be employed to inhibit the activity of endogenous alkaline phosphatase in the specimen. The reaction may be measured by kinetic or by endpoint methods.

Detection of antibodies: DIAG. 3(e)-(g).

Unlabelled antigen (Ag*) reactive with the antibody of interest are bound to a solid phase (microtiter plate, dipstick, membrane filter matrix). Antibody containing specimen is incubated in the presence of the unlabelled antigen (Ag*) for a sufficient length of time to accomplish binding. Unbound antibody may or may not be required to be removed from the matrix by washing.

Two sets of antibodies, each against the antibody of interest, one set conjugated to label 1 ($Ab-L_1$) and the other set conjugated to label 2 ($Ab-L_2$) are permitted to bind with antibody bound to the solid phase bound antigen. The formation of the ternary complex comprised of [$Ab-L_1-(Ab-L_2)-Ab-Ag^*$ -solid phase] which brings the labels into proximity with one another. The signal producing reaction is significantly greater in the ternary complex as opposed to that of occurring in free solution between the labels. The amount of signal ($S_d$) is proportional to the amount of antibody present in the sample and is a measure of the concentration of the antibody in the specimen. A scavenger (X) may be added to remove $S_2$ produced by the action of label 1 in free solution in order to diminish the background signal. Unbound labelled antibodies may be removed by washing prior to the addition of substrate to reduce background signal.

EXAMPLE: Antigens prepared from detergent inactivated, heat inactivated or recombinant produced antigens of HTLV-III are immobilized to a solid phase (microtiter plate or membrane). Goat anti-human antibodies or their fragments are separately conjugated to urease and alkaline phosphatase. Specimen containing antibodies to HTLV-III is brought into contact with the immobilized antigen and permitted to bind. Washing may be performed but is not essential.

The antibody-enzyme conjugates are brought into contact with the formed solid phase complex. The antibody-enzyme conjugates may be removed by washing but this is not essential. Urea and p-nitrophenyl phosphate substrates are added. The presence of antibodies to HTLV-III is indicated by the increase in absorbance measured at 405nm by comparison with the assay performed in the absence of antibodies to HTLV-III. Weakly acidic buffer may be added to scavenge the ammonia produced by the action of urease on urea in free solution. Levamisole may be added to inhibit the endogenous alkaline phosphatase activity of the specimen. The reaction may be measured by kinetic or endpoint methods.

Alternate method of detecting Antibodies: DIAG. 3(d).

An antibody binding moiety with affinity for the Fc part of the immunoglobulin such as protein A (PA) is bound to a solid phase. Specimen containing the antibodies of interest is added and permitted to incubate and bind to the protein A bound to the solid phase. All antibodies able to bind to protein A are bound.

Two sets of labelled antigen ($Ag-L_1$ and $Ag-L_2$) capable of binding to are permitted to incubate with the Ab-protein A - solid phase complex. The labelled antigens bind to the antibody of interest bound to protein A forming a ternary complex comprised of [$Ag-L_1-(Ag-L_2)$-AB-Protein A-solid phase] which brings the labels $L_1$ and $L_2$ into proximity. The signal producing reaction is significantly greater in the vicinity of the ternary complex as opposed to that occurring in free solution between the labels $L_1$ and $L_2$. The amount of signal ($S_d$) produced in the ternary complex is proportional to the amount of antibody present in the sample and is a measure of the concentration of the antibody in the specimen. A scavenger (X) may be added to remove $S_2$ produced by the action of label 1 in free solution in order to diminish the background signal. Unbound labelled antigens may be removed by washing prior to the addition of substrate to reduce the background.

EXAMPLE: Protein A or Protein G is bound to a solid phase (microtiter plate or membrane). Antigens from detergent, heat inactivated, or recombinant products of HTLV-III are conjugated to urease and alkaline phosphatase. Specimen containing antibodies to HTLV-III is brought into contact with the solid phase and the IgG in the specimen is bound by the immobilized protein A or protein G. The unbound portion of the specimen may be removed by washing but this is not essential.

Stoichiometrically equivalent quantities of the antigen-enzyme conjugate are brought into contact with the captured IgG from the specimen. Unbound antigen-enzyme conjugate may be removed by washing but this is not essential. Urea and p-nitrophenyl phosphate substrates are incubated with the assembled reactants. If the specimen contains IgG antibodies to HTLV-III, an increase in the absorbance measured at 405nm compared to that produced in the absence of IgG antibodies to HTLV-III will be observed. If washing is omitted, any antibodies to HTLV-III will be detected. Weakly acidic buffer may be added to scavenge ammonia produced by the actions of urease on urea produced in free solution. The reaction may be measured by kinetic or endpoint methods. Levamisole may be added to inhibit the endogenous alkaline phosphatase activity of the specimen.

Solid Phase Method For Detecting Or Measuring An Antigen Or An Antibody To An Antigen Jointly And Simultaneously Or Independently And Separately

DIAG. 4. is a schematic representation of solid phase methods for detecting or measuring an antigen or an antibody to the antigen jointly and simultaneously or individually and separately.

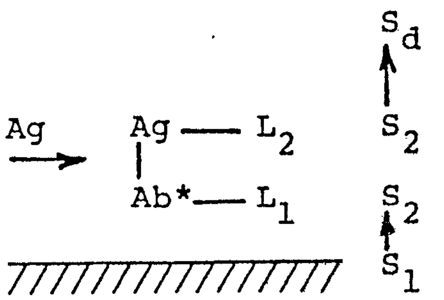


(a)

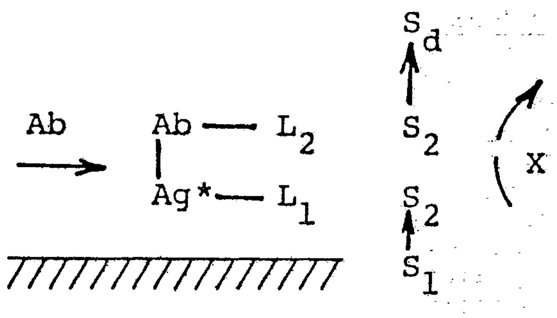


(b)

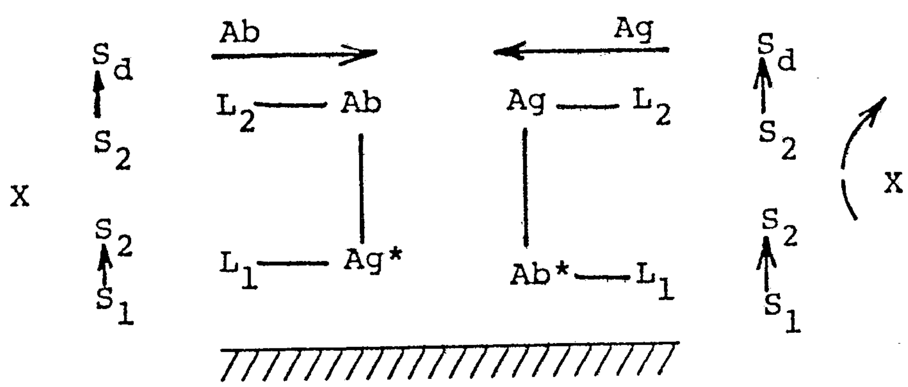


(c)

Where the symbols have the same meaning as in DIAG. 2 and,
Ab__> symbolizes unlabelled antibody in competition with Ab-$L_2$ for binding to Ag-$L_1$;
Ag__> symbolizes unlabelled antigen in competition with Ag-$L_1$ for binding to Ab-$L_2$;
* symbolizes the binding to a solid phase.

Assay for antigen: DIAG. 4(a).

Antibodies conjugated to label 1 (Ab-$L_1$) are bound to a solid phase. Specimen containing antigen capable of binding to the labelled antibodies bound to the solid phase are added and permitted to bind to the labelled antibodies. Antigen conjugated to label 2 (Ag-$L_2$) are next incubated with the specimen. The interaction between the first label and the second label produces a detectable signal ($S_d$). This signal is generated by the action of the first label or a first substrate ($S_1$), followed by the action of the second label on a second substrate ($S_2$) which was produced in the first reaction.

When unlabelled antigen is bound to the antibody conjugated to label 1 bound to the solid phase, antigen conjugated to label 2 is prevented from binding with the antibody. A diminution of signal is observed compared to that obtained in the absence of unlabelled antigen. The amount of diminution in signal is proportional to the amount of unlabelled antigen in the specimen and is a measure of the amount of unlabelled antigen present in the specimen.

Assay for antibody: DIAG. 4(b).

Antigen conjugated to label 1 (Ag-$L_1$) is bound to a solid phase. Specimen containing antibodies capable of binding to the labelled antigen bound to the solid phase are added and permitted to bind to the labelled antigen. Antibodies conjugated to label 2 (Ab-$L_2$) are next incubated with the specimen. The interaction between the first label and the second label produces a detectable signal ($S_d$). This signal is generated by the action of the first label or a first substrate ($S_1$), followed by the action of the second label on a second

substrate ($S_2$) which was produced in the first reaction.

When unlabelled antibody is bound to the antigen conjugated to label 1 bound to the solid phase, antibody conjugated to label 2 is prevented from binding with the labelled antigen. A diminution of signal is observed compared to that obtained in the absence of unlabelled antibody. The amount of diminution in signal is proportional to the amount of unlabelled antibody in the specimen and is a measure of the amount of unlabelled antibody present in the specimen.

Simultaneous assay for antigen or antibody: DIAG. 4(c).

Antibodies conjugated to label 1 (Ab-$L_1$) and antigen conjugated to label 1 (Ag-$L_1$) are bound to a solid phase. Specimen containing antigen capable of binding to the labelled antibodies bound to the solid phase and or antibodies capable of binding to the labelled antigens bound to the solid phase are added and permitted to bind. Antigen and antibody conjugated to label 2 (Ag-$L_2$ and Ab-$L_2$) are next incubated with the specimen. The interaction between the first label and the second label produces a detectable signal ($S_d$). This signal is generated by the action of the first label or a first substrate ($S_1$), followed by the action of the second label on a second substrate ($S_2$) which was produced in the first reaction.

When unlabelled antigen is bound to the antibody conjugated to label 1 bound to the solid phase, antigen conjugated to label 2 is prevented from binding with the antibody. A diminution of signal is observed compared to that obtained in the absence of unlabelled antigen. The amount of diminution in signal is proportional to the amount of unlabelled antigen in the specimen and is a measure of the amount of unlabelled antigen present in the specimen.

When unlabelled antibody is bound to the antigen conjugated to label 1 bound to the solid phase, antibody conjugated to label 2 is prevented from binding with the labelled antigen. A diminution of signal is observed compared to that obtained in the absence of unlabelled antibody. The amount of diminution in signal is proportional to the amount of unlabelled antibody in the specimen and is a measure of the amount of unlabelled antibody present in the specimen.

If either unlabelled antigen or antibody is present in the specimen the decrease in signal ($S_d$) is indicative of the presence of one or the other or both species being present in the specimen.

EXAMPLE: Antigens from HTLV-III are conjugated to urease and antibodies or their fragments reactive with HTLV-III are conjugated to urease. Antigens from HTLV-III are conjugated to alkaline phosphatase and antibodies or their fragments are conjugated to alkaline phosphatase.

Antibody conjugated to alkaline phosphatase is immobilized to a solid phase (microtiter plate or membrane). A stoichiometric amount of antigen conjugated to urease is added in the presence of specimen containing HTLV-III antigens. Urea and p-nitrophenyl phosphate substrates are added. The presence of HTLV-III antigens is indicated by a decrease in the signal obtained in their absence from the specimen. The reaction may be monitored kinetically or by the endpoint method at 405 nm. Weakly acidic buffer is added to scavenge ammonia produced by the action of urease on urea in free solution. Levamisole may be added to inhibit the endogenous alkaline phosphatase activity of the specimen.

Alternatively, antigen conjugated to alkaline phosphatase may be immobilized to solid phase, and the above reactions conducted with antibodies against HTLV-III conjugated to urease in the presence of specimen containing antibodies to HTLV-III. The presence of antibodies to HTLV-III is indicated by a decrease in the signal from that obtained in the absence of antibodies to HTLV-III in the specimen. A weakly acidic buffer may be employed to scavenge the ammonia produced by the action of urease on urea in free solution. Levamisole may be added to inhibit the activity of endogenous alkaline phosphatase in the specimen. The reaction may be measured kinetically or by the endpoint method.

Both alkaline phosphatase conjugated antibodies or their fragments to HTLV-III and antigens from HTLV-III conjugated to alkaline phosphatase may be immobilized to the solid phase permitting the above reactions to be conducted simultaneously with either the presence of antibodies to HTLV-III antigens, or both indicated by a decrease in signal compared to a specimen containing neither. Weakly acidic buffer may be added to scavenge ammonia produced by the action of urease on urea in free solution. Levamisole may be added to inhibit the activity of endogenous alkaline phosphatase in the specimen. The reaction may be measured by kinetic or endpoint methods.

Method For Measurement Of Nucleotide Sequence In Free Solution Or Bound To Solid Phases

DIAG. 5. is a schematic representation of detection of DNA or RNA sequence either in free solution or bound to solid phases or in situ.

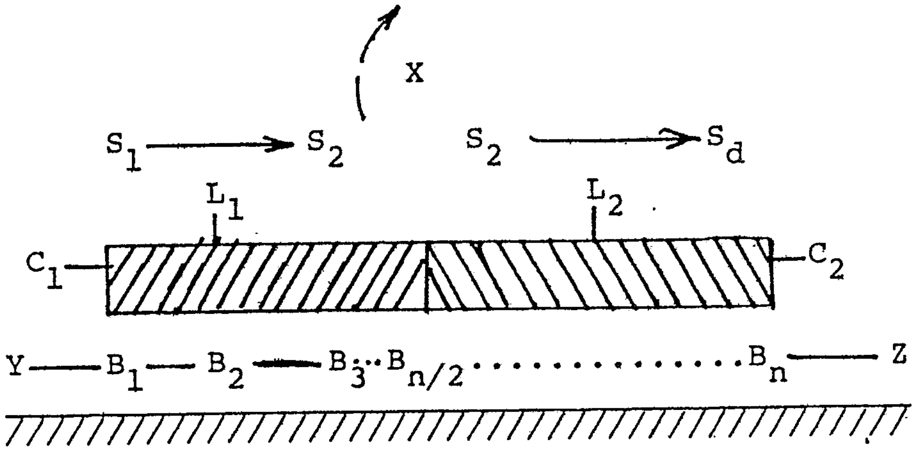


(a)

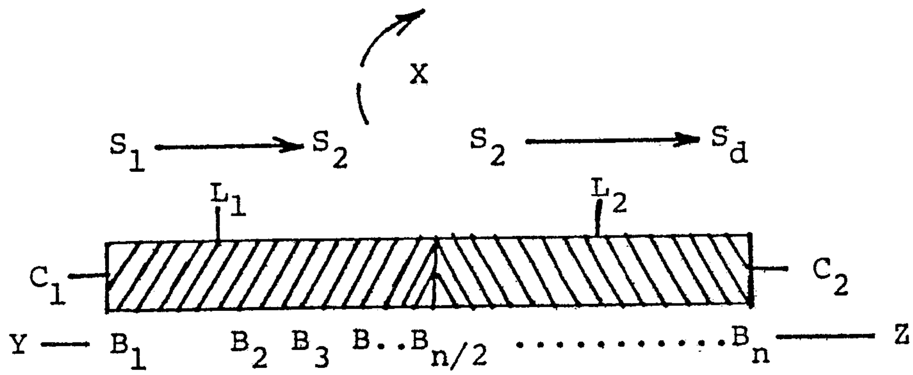


(b)

Where B symbolizes individual nucleotides and,
Bn symbolizes the location of a nucleotide in a sequence;
Y- symbolizes antecedent nucleotide sequence;
Z- symbolizes the postcedent nucleotide sequence;

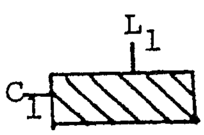


symbolizes the sequence conjugated to label 1 complimentary to the sequence Bl-Bn/2;

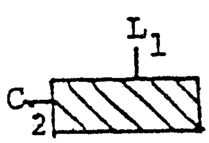


symbolizes the sequence conjugated to label 2 complimentary to the sequence Bn/2-Bn;
...NsNsNs..: symbolizes the nucleotide sequence of interest;
...NcNcNc... symbolizes the complimentary sequence to NsNsNs...;
A-A-L1 symbolizes poly adenosine conjugated to label 1;
A-A-L2 symbolizes poly adenosine conjugated to label 2;
...T-T-T... symbolizes the poly thymidine tails added by the action of terminal deoxynucleotide transferase;
3′ symbolizes the 3′-OH terminus of a sequence;
5′ symbolizes the 5′-OH terminus of a sequence;
and the remaining symbols retain their previous usage.

It is frequently important, both in a research setting and in a clinical setting, to determine if a particular nucleotide sequence is present in a specimen. There are a number of situations in which this is desirable. The screening of a plasmid library in order to ascertain whether or not a cloned sequence has been inserted properly into the plasmid gene sequence in the process of constructing a synthetic clone to express a particular gene or gene sequence of interest is such a case. The determination of the presence of a gene sequence in Dot blot hybridization for either DNA or RNA, the location of a particular gene within genomic material, or the determination of the fate of such a gene in certain biological situations constitute other situations in which sequence identity may be desirable. In certain instances, it may be desirable to determine the expression of particular mRNA's by the use of cDNA probes. It may be desirable to determine the identity of a particular pathogenic organism by establishing the presence of a genetic sequence unique to the pathogen under conditions where culture may not be possible or the constraints of time may render the results of a culture untimely and hence inadequate.

The use of complimentary sequences to identify the presence of particular gene sequences is widely known in molecular biology. A wide array of techniques have been developed that posses the requisite sensitivity to detect a gene sequence. The methods typically employ the use of radioactive isotopes usually $32_p$ which has a very short half-life of only 14 days. The present invention embodies a method in which nucleotides coupled to enzymes provide a method of distinguishing between complimentary material which is bound in the reaction from that which remains unbound. In many instances background can cause erroneous results or yield results of insufficient clarity to make the desired determination. Most techniques also require two days for their performance.

The present invention possesses the ability to discriminate between the presence or absence of a particular gene sequence in a specimen but has the ability to do so without the requisite separation required by other methods, and may be performed in a significantly shorter time. Further, the invention does not require the use of radioactive isotopes with the handling and disposal problems associated with their use.

Two complimentary sequences to the gene of interest are constructed by the employment of synthetic, recombinant, or natural methods. The two complimentary sequences are constructed in such a manner that the first construct is complimentary to the first half (or segment) of the gene, or sequence of interest and the second construct is complimentary to the second half (or segment) of the gene, or sequence of interest. Rather than employing synthetic methods an actual complimentary sequence may be recovered from the gene of interest and treated in such a manner that two fragments complimentary to continuous sequences are obtained. In the instances where the protein sequence is known a number of probes can be prepared to accommodate the degeneracy of the gene code for individual amino acids i.e. a family of probes may be constructed.

The first probe is labelled by means of conjugating it to the first enzyme of a pair of enzymes which catalyze a coupled reaction. The second probe is labelled by conjugation with the second enzyme which catalyzes the second reaction of the sequential reactions.

EXAMPLE: A length of phage DNA nucleotides in single stranded form containing a known or suspected linear sequence (target sequence) of nucleotides is bound to a solid phase. A first sequence of nucleotides complimentary to the first half of the target sequence is conjugated to urease and a second sequence of nucleotides complimentary to the remaining half of the target sequence of nucleotides is conjugated to alkaline phosphatase. The conjugated nucleotide sequences are permitted to bind to the target sequence. The urease and alkaline phosphatase are bound in proximity to one another. Upon addition of urea and p-nitrophenyl phosphate substrates, the signal ($S_d$) produced by the concerted action of the bound labels in proximity on the substrate is substantially greater than that caused by the action of the unbound labels in free solution. The intensity of the signal measured at 405nm is proportional to the amount of target sequence present in the specimen and is a measure of the quantity of the target sequence present in the specimen. A weakly acidic buffer may be employed to scavenge ammonia produced by the action of urease on urea in free solution. Levamisole may be employed to inhibit the activity of endogenous alkaline phosphatase in the specimen. The reaction may be measured by kinetic or endpoint methods.

The same sequence of reactions can also be performed in free solution, with the intensity of the signal also being proportional to the amount of target sequence present is the specimen. A weakly acidic buffer may be employed to scavenge the ammonia produced by the action of urease on urea in free solution. Levamisole may be employed to inhibit the activity of endogenous alkaline phosphatase in the specimen. The reaction may be measured by kinetic or by endpoint methods.

Detection of nucleotide sequence by end labelling: DIAG. 5(b).

EXAMPLE: A nucleotide probe is allowed to hybridize with the complimentary sequence of interest. The unbound probe and the unbound target are broken down into mono and di nucleotides by the action of nuclease $S_1$ (E.C. 3.1.30.1). The hybridized portions of the sequence of interest are not affected by the action of this enzyme. After sufficient time has elapsed to accomplish the destruction of noncomplimentary sequences, the nuclease SI is inactivated.

Terminal deoxynucleotide transferase is added to catalyze the 3′-OH addition of a homopolymer of Thymidine triphosphate (TTP). The target sequence and the probe sequence are labelled from their 3′-OH ends with poly T tails.

Short sequences of poly Adenosine, one group conjugated to label 1, urease, and the other group conjugated to label 2, alkaline phosphatase, are added. The labelled poly A binds to the poly T tails in such a manner that the two labels, poly Adenosine conjugates, are brought into close proximity to one another. Urea and p-nitrophenyl phosphate substrates are added. The signal ($S_d$) is an increase in absorbance measured at 405nm produced by the concerted action of the enzyme conjugates (first and second labels) on their respective substrates and is proportional to the number of complimentary sequences present in the specimen and is also an indication of the presence of the sequence of interest in the specimen of interest. A scavenger (X) may be added to decrease the background due to the formation of $S_2$ in free solution. In particular, a weakly acidic buffer may be employed to scavenge the ammonia produced by the action of urease on urea in free solution. Levamisole may be employed to inhibit the activity of endogenous alkaline phosphatase in the specimen. The reaction may be measured by kinetic or by endpoint methods.

Detection Of A Receptor On A Cell Surface Or On Isolated Membranes

DIAG. 6. is a schematic representation of detection of a receptor or modulator on a cell surface or in an isolated form.

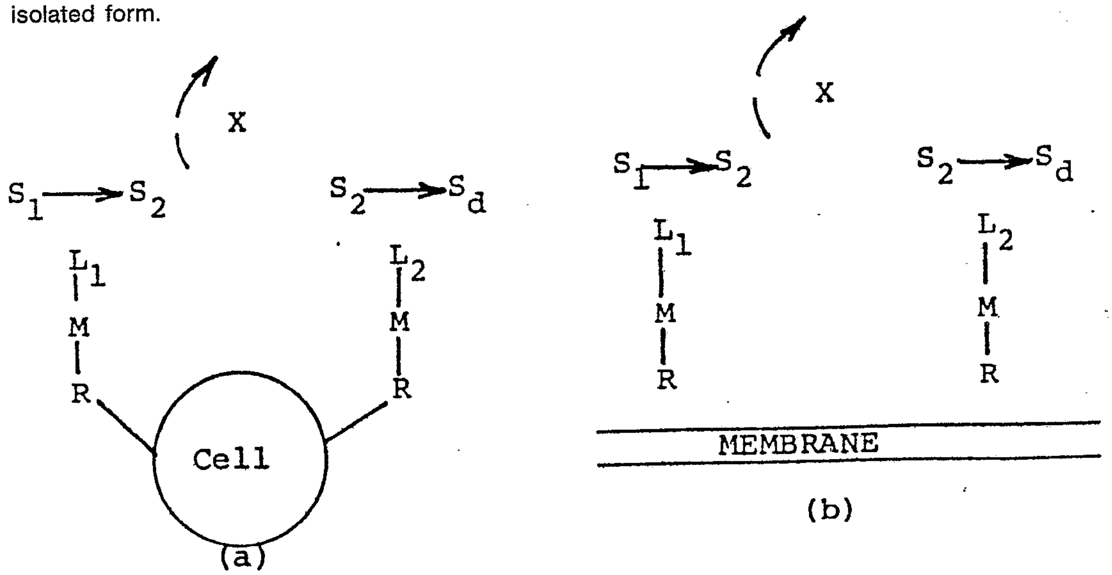


Where R symbolizes the membrane bound receptor;
$M-L_1$ symbolizes the modulator conjugated to label 1;
$M-L_2$ symbolizes the modulator conjugated to label 2;
and the remaining symbols retain their previous usage.

Detection of receptors on cells: DIAG. 6(a).

Cells containing the receptor of interest are incubated with two sets of a modulator molecule capable of binding selectively to the receptor. One set is the modulator conjugated to label 1 ($M-L_1$) and the second set is the modulator conjugated to label 2 ($M-L_2$). The labelled modulators bind to the receptors on the cell surface. The density of the receptors on the cell surface creates conditions favorable to the proximate binding of sufficient modulator conjugated to label 1 and modulator conjugated to label 2 that the signal ($S_d$) generated by the concerted action of the two labels at the cell surface is substantially greater than that occurring between the two labels in their unbound state in free solution. The intensity of the signal ($S_d$) is proportional to the quantity of receptors present in the specimen. Since the intensity of the signal is representative of the number of receptors present on a given cell, the intensity of the signal is also an indicator of the number of cells bearing the particular receptors of interest.

Detection of receptors on isolated membranes: DIAG. 6(b).

Membranes containing the receptor of interest are incubated with two sets of a modulator molecule capable of binding selectively to the receptor. One set is the modulator conjugated to label 1 ($M-L_1$) and the second set is the modulator conjugated to label 2 ($M-L_2$). The labelled modulators bind to the receptors on the membrane surface. The density of the receptors on the membrane surface creates conditions favorable to the proximate binding of sufficient modulator conjugated to label 1 and modulator conjugated to label 2 that the signal ($S_d$) generated by the concerted as action of the two labels at the cell surface is substantially greater than that occurring between the two labels in their unbound state in free solution. The intensity of the signal ($S_d$) is proportional to the quantity of receptors present in the specimen. Since the intensity of the signal is representative of the number of receptors present on a given membrane, the intensity of the signal is also an indicator of the number of the particular receptors of interest.

EXAMPLE: Epidermal growth factor (EGF) is conjugated to alkaline phosphatase and urease in two separate reactions. Cells containing the receptor for EGF (i.e. EGFr) are incubated in the presence of stoichiometrically equivalent amounts of the two EGF-enzyme conjugates. Urea and p-nitrophenyl phosphate substrates are added. The presence of receptor is indicated by an increase in absorbance measured at 405nm compared to the assay performed with cells free of the EGF receptor. A calibration curve can be prepared and the average number of EGF receptors on a cell can be measured as well as a count of the number of cells bearing the EGF receptor.

The reaction can be carried out in a similar manner on isolated cell membranes obtained from cells bearing the EGF receptor. Weakly acidic buffer may be added to scavenge the ammonia produced by the reaction of urease on urea in free solution. Levamisole may be employed to inhibit the activity of endogenous alkaline phosphatase in the specimen. The reaction may be measured by kinetic or by endpoint methods.

<u>Detection Of A Receptor Or Modulator Jointly And Simultaneously Or Individually And Separately</u>

DIAG. 7. is a schematic representation of detection of a receptor or modulator jointly and simultaneously or individually and separately.

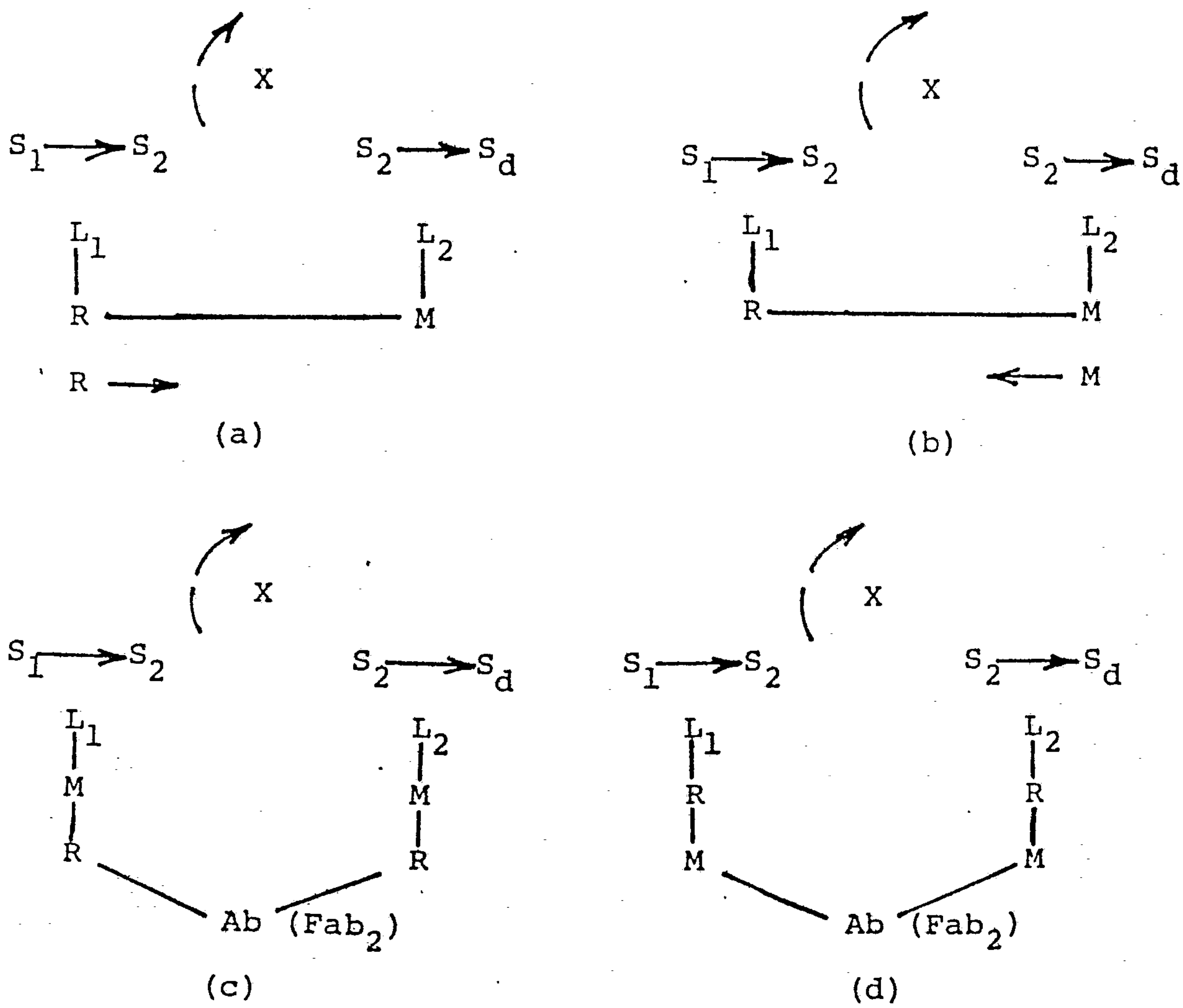


Where R-> symbolizes unlabelled receptor competing with R-$L_1$ for binding to M-$L_2$;
M-> symbolizes unlabelled modulator competing with M-$L_2$ for binding to R-$L_1$;
R-$L_1$ symbolizes receptor conjugated to label 1;
M-$L_2$ symbolizes modulator conjugated to label 2;
M symbolizes unlabelled modulator;
and the remaining symbols retain their previous usage.

<u>Detection of a receptor or modulator jointly and simultaneously: DIAG. 7(a)-(b).</u>

Stoichiometrically equivalent amounts of purified receptor conjugated to label (R-$L_1$) is allowed to bond with modulator conjugated to label 2 (M-$L_2$) in the presence of specimen containing unlabelled receptor ($R_{->}$) or modulator ($M_{->}$). The unlabelled moieties compete with the labelled moieties for binding to one another ($R_{->}$ with $R-L_1$, and $M_{->}$ with $M-L_2$). The formation of non-productive binding ($R_{->}-M-L_2$ and $R-L_1-M_{->}$) complexes as opposed to the productive complex ($R-L_1-M-L_2$) causes a diminution in signal ($S_d$), as opposed to that occurring in the absence of unlabelled receptor or modulator in the specimen. The amount of decrease in signal is proportional to the concentration of the receptor or modulator or both being present in the specimen. When the specimen contains modulator only, the decrease in signal is proportional to the quantity of the modulator in the specimen. When the specimen contains receptor only, the decrease in signal is proportional to the quantity of receptor in the specimen. When the specimen contains both receptor and modulator the decrease is proportional to the concentration of both receptor and modulator. The decrease in signal also serves to indicate the presence of either one or the other or both being present in the specimen.

EXAMPLE: Isolated receptors to epidermal growth factor (EGFr) are conjugated to urease. EGF is conjugated to alkaline phosphatase.

Stoichiometrically equivalent amounts of $EGF_r$-urease conjugate and the EGF-alkaline phosphatase conjugate are incubated in the presence of specimen containing free $EGF_r$ or EGF. The presence of either $EGF_r$ or EGF is indicated by the decrease in signal measured by absorbance at 405nm compared to the reaction carried out in the absence of free $EGF_r$ or EGF. Weakly acidic buffer is added to scavenge ammonia produced by the reaction of urease on urea in free solution. Levamisole may be employed to inhibit the activity of endogenous alkaline phosphatase in the specimen. The reaction may be measured by kinetic or by endpoint methods.

Detection of a receptor: DIAG. 7(c).

Unlabelled antibodies (Ab) to a receptor (R) are incubated together and permitted to form an immune complex, ($Ab-R_2$). Two sets of labelled modulator, one set conjugated to label 1 ($M-L_1$) and the second set conjugated to label 2 ($M-L_2$), are added to the formed complex. Because of the bivalent nature of antibodies, a significant number of [$AbR_2(M-L_1)-M-L_2$] complexes are formed. The activity of the labels $L_1$ and $L_2$ within this complex produces a signal ($S_d$) significantly greater than that produced by the reaction between the unbound modulators bearing the labels. A scavenger (X) may be added to reduce the signal contribution from the reaction taking place between the unbound modulators in free solution. The amount of signal ($S_d$) produced in the productive complex is proportional to the quantity of receptors present in the specimen, and is a measure of the quantity of receptor in the specimen. The antibodies employed to bind the receptors may also be bound to a solid phase rather than in free solution.

EXAMPLE: Epidermal growth factor is conjugated separately to urease and alkaline phosphatase.

Antibodies to the epidermal growth factor receptor ($EGF_r$) are incubated with a specimen containing $EGF_r$. Stoichiometrically equivalent amounts of EGF-enzyme conjugates are added. Urea and p-nitrophenyl phosphate substrates are added. In the presence of $EGF_r$ an increase in the absorbance measured at 405nm is observed compared to that obtained performing the identical reaction with a specimen free of the $EGF_r$. Weakly acidic buffer may be added to scavenge ammonia produced by the action of urease on urea in free solution. Levamisole may be employed to inhibit the activity of endogenous alkaline phosphatase in the specimen. The reaction may be measured by kinetic or by end-point methods.

By a similar method EGF can be measured where in $EGF_r$ is conjugated separately to urease and alkaline phosphatase. Antibodies or their $Fab_2$ fragments reactive with EGF are added to specimen containing EGF. Stoichiometrically equivalent amounts of conjugated $EGF_r$-enzyme conjugates are added. Urea and p-nitrophenyl phosphate substrates are added. In the presence of EGF an increase in the absorbance measured at 405nm is observed compared to specimen free of EGF. Weakly acidic buffer may be added to scavenge ammonia produced by the reaction of urease on urea in free solution. Levamisole may be employed to inhibit the activity of endogenous alkaline phosphatase in the specimen. The reaction may be measured by kinetic or by end-point methods.

Detection of a modulator: DIAG. 7(d).

Unlabelled antibodies (Ab) to a modulator (M) are incubated together and permitted to form an immune complex ($Ab-M_2$). Two sets of labelled receptor, one set conjugated to label 1 ($R-L_1$) and the second set conjugated to label 2 ($R-L_2$), are added to the formed complex. Because of the bivalent nature of antibodies, a significant number of [$Ab-M_2(R-L_1)-R-L_2$] complexes are formed. The activity of the labels $L_1$ and $L_2$ within this complex produces a signal ($S_d$) significantly greater than that produced by the reaction between the unbound receptors bearing the labels. A scavenger (X) may be added to reduce the signal contribution from the reaction taking place between the unbound receptors in free solution. The amount of signal ($S_d$) produced in the productive complex is proportional to the quantity of modulators present in the specimen, and is a measure of the quantity of modulator in the specimen. The antibodies employed to bind the modulators may also be bound to a solid phase rather than in free solution.

## Detection Of A Lectin Or An Entity Capable Of Specifically Binding To A Lectin

DIAG. 8. is a schematic representation of detection of a lectin or an entity capable of specifically binding to it.

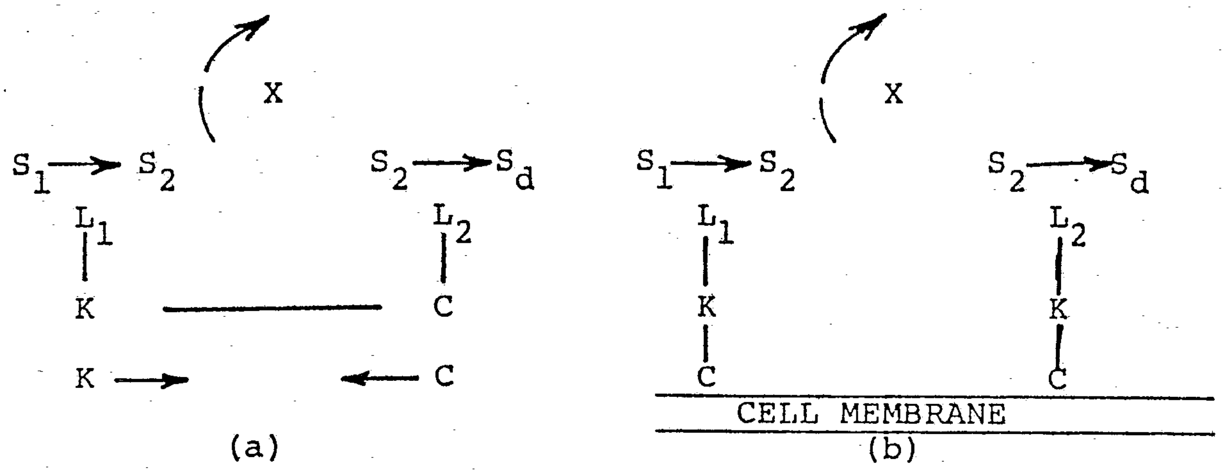


Where K-> symbolizes the unconjugated lectin in competition with K-$L_1$;
C-> symbolizes the unconjugated lectin binding entity in competition with C-$L_2$;
K-$L_1$ symbolizes the lectin conjugated to label 1;
K-$L_2$ symbolizes the lectin conjugated to label 2;
C-$L_2$ symbolizes the lectin binding entity conjugated to label 2;
K symbolizes unconjugated lectin;
C symbolizes unconjugated lectin binding entity;
and the remaining symbols retain their previous usage.

In free solution: DIAG. 8(a).

Stoichiometric binding equivalents of lectin conjugated to label 1 (K-$L_1$) and lectin binding entity conjugated to label 2 (C-$L_2$) are incubated in the presence of specimen containing unconjugated lectins (K->) or lectin binding entity (C->). The unlabelled moieties compete with their respective labelled moieties for binding with the binding partner.(K-> with K-L1, and C-> with C-L2). The formation of non-productive binding complexes (K->-C-L2 and K-L1-C->) as opposed to productive complexes (K-L1-C-L2) causes a diminution in signal (Sd) as opposed to that occurring in the absence of unlabelled lectin or unlabelled lectin binding entity in the specimen. The amount of decrease in signal is proportional to the concentration of the lectin binding entity or both being present in the specimen. When the specimen contains lectin only, the decrease in signal is proportional to the quantity of the lectins in the specimen. When the specimen contains lectin binding entity only, the decrease in signal is proportional to the quantity of the lectin binding entity in the specimen. When the specimen contains both lectin and lectin binding entity, the decrease in signal is proportional to the concentration of both lectin and lectin binding entity in the specimen. The decrease in signal also serves to indicate the presence of either one or the other or both being present in the specimen.

Detection of lectin binding entity on cells: DIAG. 8(b).

Cells containing the lectin binding entity (C) are reacted with two sets of labelled lectins, one set composed of the lectin conjugated to label 1 (K-$L_1$) and the other set composed of lectin conjugated to label 2 (K-$L_2$). The labelled lectins bind to the lectin binding entities on the cell surface. The distribution of the lectin binding entities on the cell surface creates conditions favorable to the proximate binding of sufficient labelled lectins (K-$L_1$ and K-$L_2$), that the signal generated by the concerted action of the two labels at the cell surface is substantially greater than that occurring between the two labelled lectin in their unbound state in free solution. The intensity of the signal ($S_d$) produced is proportional to the quantity of lectin binding entities on the cells. Since the intensity of the signal is representative of the number of receptors present on a given cell, the intensity of the signal is also an indicator of the number of cells bearing the particular lectin binding entity of interest.

EXAMPLE: Asialo GM1 ganglioside is conjugated to alkaline phosphatase. The lectin peanut agglutinin from Arachis hypogaea is conjugated to urease. Stoichiometrically equivalent amounts of the lectin-enzyme and ganglioside-enzyme conjugates are incubated in the presence of either free lectin or free asialo GM1 ganglioside. The presence of either free lectin, free asialo GM1 ganglioside or both, will cause a decrease in the absorbance measured at 405nm compared to that produced by the equivalent reaction performed in the absence of free lectin or ganglioside or both. Weakly acidic buffer may be added to scavenge ammonia produced by the action of urease on urea in free solution. Levamisole may be employed to inhibit the activity of endogenous alkaline phosphatase in the specimen. The reaction may be measured by kinetic or by end-point methods.

The peanut agglutinin is conjugated separately to urease and alkaline phosphatase. Cells containing the asialo GM1 ganglioside are incubated in the presence of stoichiometrically equivalent amounts of the lectin-enzyme conjugates. Urea and p-nitrophenyl phosphate substrates are added. The presence of cells containing the asialo GM1 ganglioside is indicated by the increase in absorbance at 405nm observed compared to an identical reaction performed of cells lacking the asialo GM1 ganglioside. Weakly acidic buffer may be added to scavenge ammonia produced by the action of urease on urea in free solution. Levamisole may be employed to inhibit the activity of endogenous alkaline phosphatase in the specimen. The reaction may be measured by kinetic or by end-point methods.

Detection Of An Antigenic Determinant On Cells

DIAG. 9. is a schematic representation of detection of a cell marker by means of an antibody specifically binding to it.

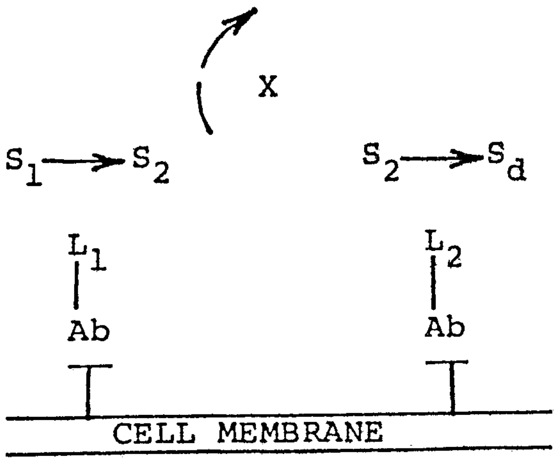


Where T symbolizes a unique antigenic determinant on the cells of interest;
Ab-$L_1$ symbolizes antibody conjugated to label 1;
Ab-$L_2$ symbolizes antibody conjugated to label 2;
and the remaining symbols retain their previous usage.

Cells containing the antigenic entity (T) are reacted with two sets of labelled antibodies, one set composed of the antibody conjugated to label 1 (Ab-$L_1$) and the other set composed of antibody conjugated to label 2 (Ab-$L_2$); see DIAG. 9. The labelled antibodies bind to the antigenic entities on the cell surface. The distribution of the antigenic entities on the cell surface creates conditions favorable to the proximate binding of sufficient labelled antibodies (Ab$L_1$ and Ab-$L_2$), that the signal generated by the concerted action of the two labels at the cell surface is substantially greater than that occurring between the two labelled antibodies in their unbound state in free solution. The intensity of the signal ($S_d$) produced is proportional to the quantity of antigenic entities on the cells. Since the intensity of the signal is representative of the number of antigenic species present on a given cell reactive with the antibodies, the intensity of the signal is also an indicator of the number of cells bearing the particular antigenic species reactive with the antibodies.

EXAMPLE: Antibodies (polyclonal, monoclonal or their fragments or chimeric constructs) reactive with carcinoembryonic antigen (CEA) are conjugated separately to urease and alkaline phosphatase. Cells bearing CEA marker are incubated with stoichiometrically equivalent amounts of the enzyme conjugated antibodies. Urea and p-nitrophenyl phosphate substrates are added. The presence of cells bearing the CEA marker are indicated by the increase in absorbance measured at 405nm compared to an identical reaction performed with cells not bearing the CEA marker. Weakly acidic buffer may be added to scavenge ammonia produced by the action of urease on urea in free solution. Levamisole may be employed to inhibit the activity of endogenous alkaline phosphatase in the specimen. The reaction may be measured by kinetic or by end-point methods.

The reaction may be performed on sections of tissue obtained in autopsy, biopsy or surgically removed, after fixation for histopathological examination. When immunohistochemistry is performed, naphthol AS phosphate and Fast Red TR are employed as substrate for alkaline phosphatase in place of p-nitrophenyl phosphate.

Selective Cell Toxicity

DIAG. 10. is a schematic depiction of selective toxicity achievable by the invention.

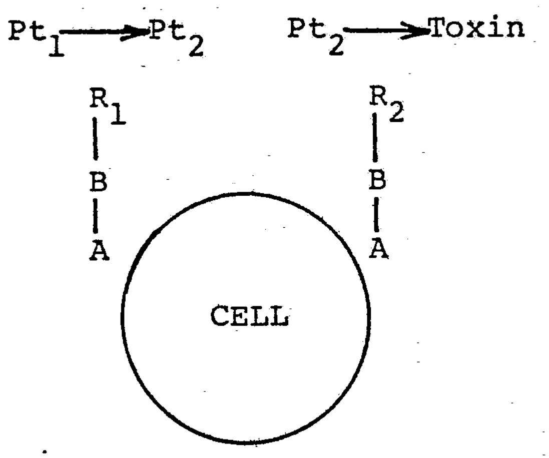


Where A symbolizes a moiety capable of binding B located on the target cells;
B-$R_1$ symbolizes binding moiety conjugated to reactive center 1;
B-$R_2$ symbolizes binding moiety conjugated to reactive center 2;
$Pt_1$ symbolizes the substrate acted on by $R_1$ to give $Pt_2$;
$Pt_2$ symbolizes the substrate produced by the action of $R_1$ on $Pt_1$, which is acted on by $R_2$ to give Toxin;
Toxin symbolizes the product of the action of R2 on Pt2, which is capable of causing cell death or impairment of cellular metabolism.

The invention also embraces the ability to achieve local levels of toxins in the region proximate to the bound enzymes catalyzing a sequential interaction; see DIAG. 10. Because the binding is specific in nature, selected cells, tissues, viruses, bacteria, fungi other microorganisms and entities can be selectively targeted for destruction while sparring the surrounding viable and functional tissues. The selectivity is accomplished by choosing a binding reaction isolated to the target of interest and by labelling one set of the bound moiety with the first reactive center (B-$R_1$), which catalyzes the first reaction of a sequential reaction capable of producing a toxic substance when acting in concert with the second reactive center of the sequential pair. A second set of the bound moiety is labelled with the second reactive center (B-$R_2$), which catalyzes the second reaction of the sequential pair. The sequential reaction ($PT_1$->$PT_2$->Toxin) of the first and second reactive center labels is capable of producing a toxic substance which is capable of causing cell death or metabolic impairment of the target entity. The binding of both labelled sets of the moiety to the target places the reactive centers in molecular proximity to one another. Exposure of the reactive centers to the appropriate substance(s) or condition(s), which is(are) benign unless acted upon by the reactive centers in concert, causes a local formation of a toxic substance (Toxin) capable of causing cell death or metabolic impairment of the target. In those areas where the enzymes are not in molecular proximity to one another little if any toxic substance is produced and if produced the levels are insufficient to cause cell death or metabolic impairment to non-targeted entities.

EXAMPLE: Epidermal growth factor (EGF) is separately conjugated to D-amino acid oxidase and to horse radish peroxidase. The EGF-enzyme conjugates are incubated with human fibroblast cells. Unbound conjugates are removed by washing and centrifugation at 4°C. Upon the addition of D-alanine and iodide ion as substrates the cells are permitted to incubate at 37°C. Within 24 hours there is a considerable decline in the number of viable cells in the culture compared to a culture treated in the same manner to which no substrate was added. Cell death may also be caused by employing the same enzyme pair but omitting the iodide. This permits the peroxidase to initiate free radical formation in the cell membrane lipids promoting the process of lipid peroxidation resulting in damage to the cell membrane to such an extent that cellular viability is lost.

Cytotoxicity may be accomplished by employing either the enzyme pair glucose oxidase (E.C. 1.1.3.4) and horse radish peroxidase (E.C. 1.11.1.7) or the pair D-amino acid oxidase (E.C. 1.4.3.3) and horse radish peroxidase. The enzymes are coupled to the entity capable of specific and selective interaction with the target cells.

By using the hydrogen peroxide produced by either glucose oxidase or D-amino acid oxidase as a substrate, horse radish peroxidase can catalyze the oxidation of bromide ion (Br-) or iodide ion (I-) to bromine ($Br_2$) or iodine ($I_2$) respectively. Bromine and iodine are strong oxidizing agents and their ability to kill cells is widely known. Because the production of bromine or iodine from their precursor ions by the action of peroxidase requires the presence of glucose oxidase, they are produced only when the two enzymes are in molecular proximity to one another, this is most likely to occur at the cell surface capable of participating in the binding of the entity to which the enzymes are conjugated.

In the case of D-amino acid oxidase, the enzyme becomes catalytically active when a bolus of its substrate ( a D-amino acid) is administered. This affords the opportunity to selectively initiate the cytotoxic activity of the enzyme pair. Further, the toxic substances are produced at a local site rather than systemically. The toxins are produced continuously at the target site until such time as the enzymes are separated by the demise and disassociation of the target cell, at which time the activity of the enzyme pair becomes greatly diminished if not nil. This is in contrast to current therapies based on immunotoxins, which remain toxic until degraded, and may affect other cells distant from the desired target, and the toxins employed, while extraordinarily toxic, do not create conditions of repetitious exposure to the target cells.

While only a few exemplary embodiments of this invention have been described in detail, those skilled in the art will recognize that there are many possible variations and modifications which may be made in the exemplary embodiments while yet retaining many of the novel and advantageous features of this invention. Accordingly, it is intended that the following claims cover all such modifications and variations.

## Claims

1. A method, targeting selected molecular species to produce at least one selected substance in a fluid comprising:

a) conjugating a first label to a first moiety and a second label to a second moiety, said first and second moieties complimentarily binding to complimentary entities being one of,

1) respective first and second complimentary sites on a solid phase, and

2) each other, and

3) a single entity, complimentary to both; and

(b) producing a selected substance, said substance being produced in the presence of a first substrate, by an interaction between said first label and said second label, the amount of said substance being enhanced by the close proximity of said labels.

2. An assay to determine the presence or concentration of at least one selected substance in a fluid comprising:

(a) conjugating a first label to a first moiety and a second label to a second moiety, said first and second moieties complimentarily binding to complimentary entities; and

(b) detecting a signal indicating the presence or concentration of said substance, said signal being produced in the presence of a first substrate, by an interaction between said first label and said second label, said signal being enhanced by the close proximity of said labels.

3. A nucleotide assay to determine a sequence of nucleotides in a fluid, comprising:

(a) binding a target linear sequence of DNA or RNA nucleotides in a single strand to a solid phase;

(b) conjugating a first label to a first sequence of nucleotides complementary to said first portion of said target sequence and conjugating a second label to a second sequence of nucleotides complementary to said second portion of said target sequence, said first and second sequences of nucleotides binding to said first and second portions of said target sequence; and

(c) detecting a signal produced in the presence of a first substrate, by an interaction between said first label and said second label, enhanced by the proximity to each other by said first and second portions of said target nucleotide sequence.

4. The nucleotide assay according to claim 3 wherein said target nucleotide sequence is bound to a solid phase.

5. The nucleotide assay according to claim 3, wherein said first label and said second label are each enzymes, and said signal is produced by the sequential action of said first enzyme label on or in the presence of a first substance, and said second enzyme label on or in the presence of a second substrate, said second substrate being a product of the action of said first enzyme label on said first substrate.

6. The nucleotide assay according to claim 5, wherein said signal is an increased rate of disappearance of said first substrate.

7. The nucleotide assay according to claim 5, wherein said signal is an increased rate of disappearance of said second substrate.

8. The nucleotide assay according to claim 5, wherein said signal is an increased rate of a set of cyclic reactions, in which said first substrate is regenerated by the action of said second enzyme label on or in the presence of said second substrate.

9. The nucleotide assay according to claim 5, further including the step of adding a scavenger to said fluid to scavenge any second substrate produced by any unconjugated first label in the fluid by the action of said scavenger, to prevent its reaction with the second label.

10. A process for determining the presence or concentration of a nucleotide sequence in a fluid, comprising the steps of:

(a) contacting a solution containing a target linear sequence of DNA or RNA nucleotides in a single strand, having first and second portions, with a solution containing a first sequence of nucleotides conjugated to a first label and complimentary to said first portion of said target sequence, and a second sequence of nucleotides conjugated to a second label and complimentary to said second portion of said target sequence, said first and second complimentary sequences of nucleotides binding to said first and second portions of said target sequence;

(b) contacting the fluid with a substrate for said first label, in order to form a first reaction product from said first label and said substrate;

(c) measuring a signal produced by the reaction of said second label in the presence of said first reaction product;

(d) (i) relating the signal measured in step (c) to the signal measured for a control sample prepared according to steps (a)-(c), said control sample being free of target nucleotide sequences, in order to determine the presence of the target nucleotide sequence in said fluid; or

(ii) relating the signal measured in step (c) to the signal measured for samples containing known amounts of said target nucleotide sequence prepared according to steps (a)-(c), in order to determine the concentration of said target nucleotide sequence in said fluid.

11. A nucleotide assay to determine the presence or concentration of a nucleotide sequence in a fluid, comprising:

(a) binding a probe of a DNA or RNA nucleotide sequence with a complimentary nucleotide sequence;

(b) destroying unbound probe and unbound target nucleotide sequences by action of the enzyme nuclease S1, the bound portions of said probe sequence and complimentary sequence being uneffected by the action of said nuclease, said nuclease subsequently being inactivated;

(c) adding a homopolymer of Thymidine triphosphate to the 3 ′OH end of said probe and target nucleotide sequences by action of a terminal transferase enzyme;

(d) conjugating one set of short sequences of poly Adenosine with a first label, and a second set of said poly Adenosine to a second label, said first and second sets of labeled poly Adenosine binding to said homopolymers of Thymidine triphosphate; and

(e) detecting a signal produced in the presence of a first substrate, by an interaction between said first label and said second label, enhanced by their proximity to each other by said bound sequences of poly Adenosine.

12. The nucleotide assay according to claim 11, wherein said first label and said second label are each enzymes, and said signal is produced by the sequential action of said first enzyme label on or in the presence of a first substrate, and said second enzyme label on or in the presence of a second substrate, said second substrate being a product of the action of said first enzyme label on said first substrate.

13. The nucleotide assay according to claim 12, wherein said signal is an increased rate of disappearance of said first substrate.

14. The nucleotide assay according to claim 12, wherein said signal is an increased rate of disappearance of said second substrate.

15. The nucleotide assay according to claim 12, wherein said signal is an increased rate of a set of cyclic reactions, in which said first substrate is regenerated by the action of said second enzyme label on or in the presence of said second substrate.

16. The nucleotide assay according to claim 12 further including the step of adding a scavenger to said fluid to scavenge any second substrate produced by any unconjugated first label in the fluid by the action of said scavenger, to prevent its reaction with the second label.

17. A process for determining the presence or concentration of a nucleotide sequence in a fluid, comprising the steps of:

(a) contacting the fluid containing a target nucleotide sequence of RNA and DNA in a single linear strand, with a solution of a probe complimentary nucleotide sequence, binding said target sequence and said probe complimentary sequence;

(b) Contacting the fluid with a solution of the enzyme, nuclease S1, destroying unbound target and probe sequences, said bound portions of said probe and target sequences being uneffected by the action of said nuclease S1, with subsequent inactivation of said nuclease S1;

(c) contacting the fluid with a solution containing a terminal nucleotide transferase to catalyze the 3′-OH addition of a homopolymer of Thymidine triphosphate;

(d) contacting the fluid with a solution of first and second sets of short sequences of poly Adenosine, said first set of poly Adenosine conjugating to a set of first label and said second set of poly Adenosine conjugating to a set of second label, said two sets of first and second labeled poly Adenosine binding to said homopolymers of Thymidine triphosphate;

(e) contacting the fluid with a solution of a substrate for said first label, in order to form a first reaction product from said first label and said substrate;

(f) measuring a signal produced by the reaction of said second label in the presence of said first reaction product;

(g) (i) relating the signal measured in step (f) to the signal measured for a control sample prepared according to steps (a)-(f) said control sample being free of said target nucleotide sequence, in order to determine the presence of the target sequence in said fluid; or

(ii) relating the signal measured in step (f) to the signal measured for samples containing known amounts of said target nucleotide sequence prepared according to steps (a)-(f), in order to determine the concentration of the target nucleotide sequence in said fluid.

18. A toxigenic complex on a cell membrane in a fluid, comprising:

(a) binding selected molecular moieties on said cell membrane to two sets of a binding molecule capable of selectably binding to said molecular moiety, said first set of binding molecule being conjugated to a set of first reactive center and said second set of said binding molecule being conjugated to a set of second reactive center; and

(b) producing a substance toxic to said cell by an interaction between said first reactive center and said second reactive center, enhanced by their proximity to each other by the density of said molecular moiety on said cell membrane.

19. The toxigenic complex according to claim 18 wherein said first reactive center and said second reactive center are each enzymes, and said toxin is produced by the sequential action of said first enzyme reactive center on or in the presence of a first substrate, and said second enzyme reactive center on or in the presence of a second substrate, said second substrate being a product of the action of said first enzyme reactive center on said first substrate.

20. A process for producing a toxigenic complex on a cell membrane in a fluid, comprising the steps of:

(a) contacting a fluid containing said cell, said cell having selected molecular moieties on the membrane, with a solution of two sets of binding molecule capable of selectively binding to said selected molecular moieties, said first set of binding molecule being conjugated to a set of first reactive center and said second set of binding molecule being conjugated to a set of second reactive center, said first and second sets of binding molecule binding to said molecular moieties on said membrane;

(b) contacting the fluid with a substrate for said first reactive center, in order to form a first reaction product from said first reactive center and said substrate, said second reactive center in the presence of said first reaction product producing a substance toxic to said cell.